# DEMANDE DE BREVET EUROPEEN

(11) **EP 1 027 882 A1**
(43) Date de publication de la demande: **16.08.2000**
(21) Numéro de dépôt: 00400246.5
(22) Date de dépôt: 31.01.2000
(51) Int. Cl.: A61K 7/42

(54) **Compositions photoprotectrices contenant un dérivé de benzotriazole et un composé à groupements benzoazolyle ou benzodiazolyle**

(30) Priorité: 12.02.1999 FR 9901728
(71) Demandeur: L'OREAL, 75008 Paris (FR)
(72) Inventeur: Caudau, Didier, 91570 Bievres (FR)
(74) Mandataire: Miszputen, Laurent

(57) **Abrégé**

L'invention concerne de nouvelles compositions cosmétiques à usage topique, en particulier pour la photoprotection de la peau et/ou des cheveux, caractérisées par le fait qu'elles comprennent, dans un support cosmétiquement acceptable notamment de type huile-dans-eau, (a) au moins un dérivé de benzotriazole particulier à titre de premier filtre, et (b), à titre de deuxième filtre, au moins un composé comportant au moins deux groupes benzoazolyle ou bien un composé comportant au moins un groupe benzodiazolyle; lesdits premier et second filtres étant présents dans lesdites compositions dans une proportion produisant une activité synergique au niveau des indices de protection solaire conférés.

L'invention concerne également leur application à la protection de la peau et des cheveux contre les effets du rayonnement ultraviolet.

## Description

La présente invention concerne de nouvelles compositions cosmétiques à usage topique plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément encore, elle concerne des compositions antisolaires comprenant, dans un support cosmétiquement acceptable, une association entre(a) au moins un dérivé de benzotriazole particulier à titre de premier filtre, et (b), à titre de deuxième filtre, au moins un composé comportant au moins deux groupes benzoazolyle ou bien un composé comportant au moins un groupe benzodiazolyle; lesdits premier et second filtres étant présents dans lesdites compositions dans une proportion produisant une activité synergique au niveau des indices de protection solaire conférés.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, cas filtres (et leurs quantités) étant sélectionnés en fonction de l'indice de protection recherché (l'indice de protection (IP) s'exprimant mathématiquement par le rapport du temps d'irradiation nécessaire pour atteindre le seuil érythématogène avec le filtre UV au temps nécessaire pour atteindre le seuil érythématogène sans filtre UV).

Or, à la suite d'importantes recherches menées dans le domaine de la photoprotection évoqué ci-dessus, la Demanderesse a découvert, de façon inattendue et surprenante, que la combinaison, dans des proportions comprises dans des limites bien déterminées, de deux filtres solaires particuliers et déjà connus en soi dans l'état de l'art, permettait, du fait d'un effet de synergie remarquable, d'obtenir des compositions antisolaires présentant des indices de protection nettement améliorés, et en tous cas largement supérieurs à ceux qui peuvent être obtenus soit avec l'un ou l'autre des filtres utilisé seul.

Cette découverte est à la base de la présente invention.

Ainsi, conformément à l'un des objets de la présente invention, il est maintenant proposé de nouvelles compositions cosmétiques, en particulier antisolaires, qui sont essentiellement caractérisées par le fait qu'elles comprennent, dans un support cosmétiquement acceptable, (i) un dérivé de benzotriazole particulier, à titre de premier filtre, et (ii) au moins un composé comportant au moins deux groupes benzazolyle ou un composé comportant au moins un groupe benzodiazolyle, à titre de second filtre; lesdits premier et second filtres étant présents dans lesdites compositions dans une proportion produisant une activité synergique au niveau des indices de protection solaire conférés.

La présente invention a également pour objet l'utilisation de telles compositions pour la fabrication de, compositions cosmétiques destinées à la protection de la peau et/ou des cheveux contre le rayonnement ultraviolet, en particulier le rayonnement solaire.

Un autre objet encore de la présente invention réside dans un procédé de traitement cosmétique pour la protection de la peau et/ou des cheveux contre le rayonnement ultraviolet, en particulier le rayonnement solaire, et qui consiste essentiellement à appliquer sur ces derniers une quantité efficace d'une composition conforme à l'invention.

D'autres caractéristiques, aspects et avantages de la présente invention apparaîtront à la lecture de la description détaillée qui va suivre.

Ainsi, conformément à l'un des objets de la présente invention, il est maintenant proposé de nouvelles compositions cosmétiques ou dermatologiques, en particulier antisolaires, qui sont essentiellement caractérisées par le fait qu'elles comprennent, dans un support cosmétiquement acceptable:
(a) à titre de premier filtre, au moins un dérivé de benzotriazole de formule (I) suivante: 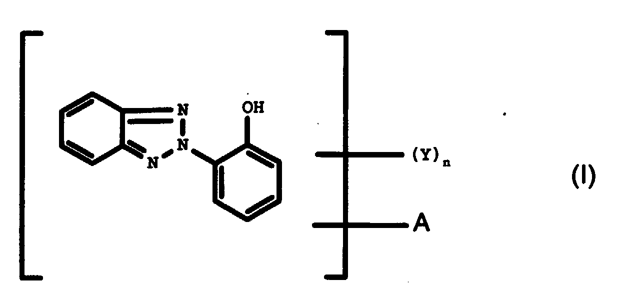 dans laquelle:
   - A désigne hydrogène ou un radical divalent -L-W-
   - Y, identiques ou différents, sont choisis parmi les radicaux alkyles en C₁-C₁₀, les halogènes, les radicaux alkoxy en C₁-C₁₀, des groupes sulfoniques, étant entendu que, dans ce dernier cas, deux Y adjacents d'un même noyau aromatique peuvent former ensemble un groupement alkylidène dioxy dans lequel le groupe alkylidène contient de 1 à 2 atomes de carbone ; sous réserve que les radicaux Y soient différents d'un groupe sulfonique quand A est différent d'hydrogène;
   - n vaut 1, 2 ou 3;
   - L est un radical divalent de formule (II) suivante: 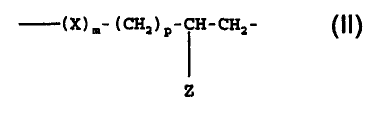 dans laquelle:
      - X représente O ou NH,
      - Z représente l'hydrogène ou un radical alkyle en C₁-C₄,
      - n est un nombre entier compris entre 0 et 3 inclusivement,
      - m est 0 ou 1,
      - p représente un nombre entier compris entre 1 et 10, inclusivement.
   - W est un radical de formule (1), (2) ou (3) suivante: 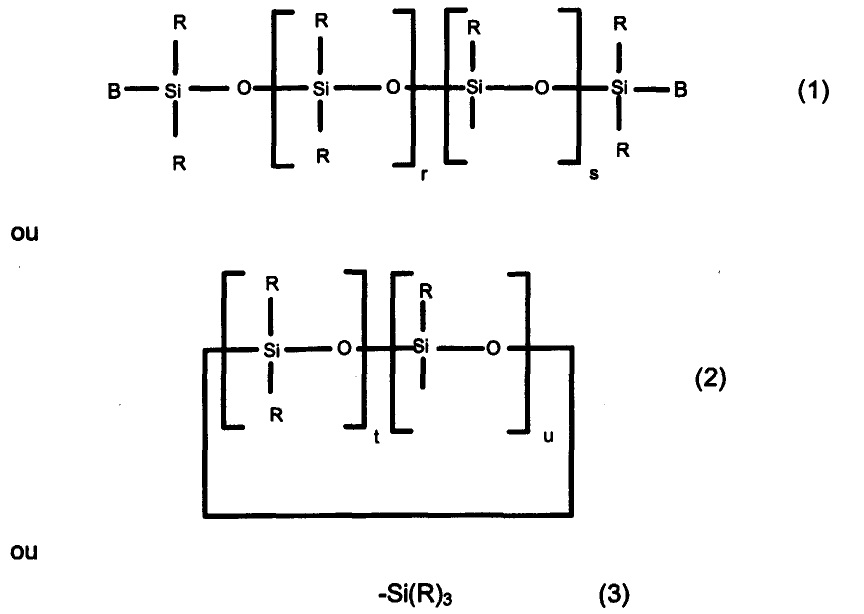 dans lesquelles:
      - R, identiques ou différents, sont choisis parmi les radicaux alkyles en C₁-C₁₀, phényle et trifluoro-3,3,3 propyle, au moins 80% en nombre des radicaux R étant méthyle,
      - B , identiques ou différents, sont choisis parmi les radicaux R et le radical V de formule suivante: 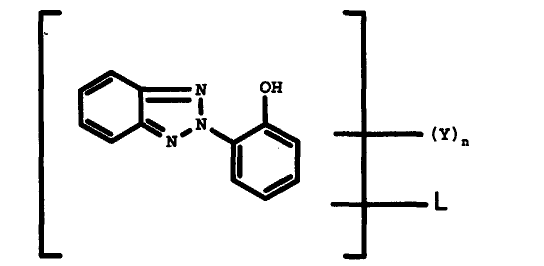 dans laquelle Y, n et L ont les mêmes significations indiquées ci-dessus;
   - r est un nombre entier compris entre 0 et 50 inclusivement, et s est un nombre entier compris entre 1 et 20 inclusivement, et si s=0 au moins l'un des deux radicaux B désigne V;
   - u est un nombre entier compris entre 1 et 6 inclusivement, et t est un nombre entier compris entre 0 et 10 inclusivement, étant entendu que t + u est égal ou supérieur à 3;
et (b) à titre de deuxième filtre, au moins un composé comportant au moins deux groupes benzazolyle par molécule ou bien un composé comportant, par molécule, au moins un groupe benzodiazolyle.

On entendra dans la description par 〈〈comportant au moins deux groupes benzoazolyle〉〉 comme comportant par molécule au moins deux groupes du type benzoxazolyle, benzothiazolyle ou benzimidazolyle.

On entendra dans la description par 〈〈comportant au moins un groupe benzodiazolyle〉〉 comme comportant par molécule un groupe du type benzodioxazolyle, benzodithiazolyle ou benzodiimidazolyle.

Les dérivés de benzotriazole de formule (I) conformes à l'invention sont des filtres déjà connus en soi. Ils sont décrits et préparés selon les synthèses indiquées dans les brevets US-4316033 et US-4328346; EP-B-0354145 et EP-B-0392883, EP-B-0660701 (faisant partie intégrante du contenu de la description).

Parmi les composés de formule (I) non siliconés où A désigne l'hydrogène, utilisables selon l'invention, on peut citer plus particulièrement
- le 2 (2 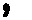 -hydroxy-5 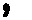 -méthylphényl)benzotriazole (n= 1 et Y= CH₃) tel que le produit vendu sous le nom UVAZOL P par la société Enichem Synth, le produit vendu sous le nom TINUVIN P par la société CIBA GEIGY;
- le 2 (2 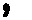 -hydroxy-3 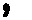 -butyl-5 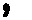 -méthylphényl)benzotriazole (n= 2 et Y= CH₃ et -C(CH₃)₃) tel que le produit vendu sous le nom UVAZOL 236 par la société Enichem Synth;
- le 2 (2 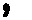 -hydroxy-5 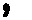 -t-octylphényl)benzotriazole (n= 1 et Y= -C(CH₃)₂-CH₂-C(CH₃)₃) tel que le produit vendu sous le nom UVAZOL 311 par la société Enichem Synth;
- le 2 (2 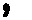 -hydroxy-3 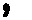 -sec-butyl-5 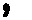 -benzènesulfonate)benzotriazole (n= 2 et Y= SO₃H ;Y= -CH(CH₃)-CH₂-CH₃) tel que le produit vendu sous le nom CIBAFAST par la société CIBA GEIGY.

Pour les composés de formule (I) siliconés où A est un radical divalent -L-W-, dans la définition des formules (1), (2) et (3) telles que définies ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, 2-éthylhexyle et ter.-octyle. Les radicaux alkyle R préférés selon l'invention sont les radicaux méthyle, éthyle, propyle, n-butyle, n-octyle et 2-éthylhexyle. Encore plus préférentiellement, les radicaux R sont tous des radicaux méthyle.

Pour les composés de formule (I) siliconés où A est un radical divalent -L-W-, on préfère mettre en oeuvre ceux où W répond à la formule (1), c'est-à-dire des diorganosiloxanes à chaîne linéaire.

Parmi les diorganosiloxanes linéaires rentrant dans le cadre de la présente invention, on préfère plus particulièrement les dérivés statistiques ou bien définis à blocs présentant au moins l'une, et encore plus préférentiellement l'ensemble, des caractéristiques suivantes:
- R est alkyle et encore plus préférentiellement est méthyle,
- B est alkyle et encore plus préférentiellement est méthyle,
- r est compris entre 0 et 15 inclusivement ; s est compris entre 1 et 5 inclusivement,
- n est non nul, et de préférence égal à 1, et Y est alors choisi parmi méthyle, ter.-butyle ou alcoxy en C₁-C₄,
- Z est hydrogène ou méthyle,
- m=0, ou [m=1 et X=O]
   - p est égal à 1.

Comme cela ressort de la formule (I) donnée ci-dessus, l'accrochage du chaînon -(X)ₘ-(CH₂)ₚ-CH(Z)-CH₂- sur le motif benzotriazole, qui assure donc le raccordement dudit motif benzotriazole à l'atome de silicium de la chaîne siliconée, peut, selon la présente invention, se faire dans toutes les positions disponibles offertes par les deux noyaux aromatiques du benzotriazole:

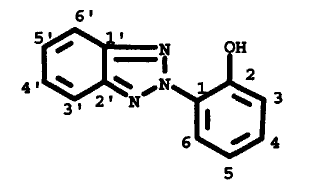

De préférence, cet accrochage se fait en position 3, 4, 5 (noyau aromatique portant la fonction hydroxy) ou 4' (noyau benzénique adjacent au cycle triazolé), et encore plus préférentiellement en position 3, 4 ou 5. Dans une forme préférée de réalisation de l'invention, l'accrochage se fait en position 3.

De même, l'accrochage du motif substituant Y peut se faire dans toutes les autres positions disponibles au sein du benzotriazole. Toutefois, de préférence, cet accrochage se fait en position 3, 4, 4', 5 et/ou 6. Dans une forme préférée de réalisation de l'invention, l'accrochage se fait en position 5.

Une famille de composés convenant particulièrement à l'invention est celle définie par la formule générale suivante: 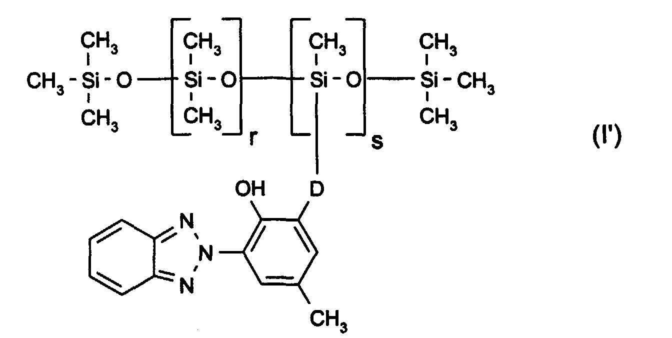 avec
0 ≤ r ≤ 15, de préférence 0 ≤ r ≤ 10
1 ≤ s ≤ 5, de préférence 1 ≤ s ≤ 3
et où D représente le radical divalent:

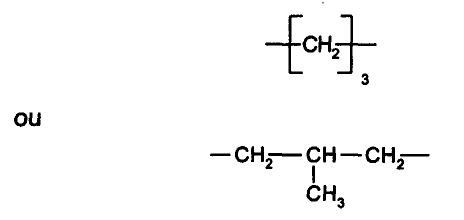

Dans une forme particulièrement préférée de réalisation de l'invention, la silicone benzotriazole répond à la formule générale (I 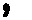 ) dans laquelle:

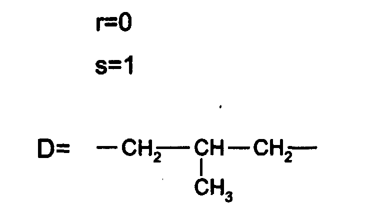

Dans une autre forme particulièrement préférée de réalisation de l'invention, la silicone benzotriazole répond à la formule générale (I 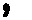 ) dans laquelle:

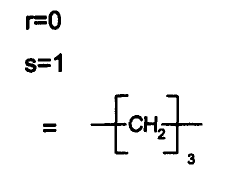

Les dérivés de benzotriazole de formule (I) peuvent être présents à des teneurs comprises entre 0,1 et 15%, de préférence entre 0,2 et 10%, en poids, toujours par rapport au poids total de la composition ; de préférence, la teneur globale du mélange entre le premier filtre solaire (i) et le deuxième filtre solaire (i) n'excède pas 15% du poids total de la composition finale.

Parmi les composés comportant au moins deux groupes benzoazolyle conformes à l'invention, on utilisera de préférence ceux répondant à la formule générale (III) suivante: 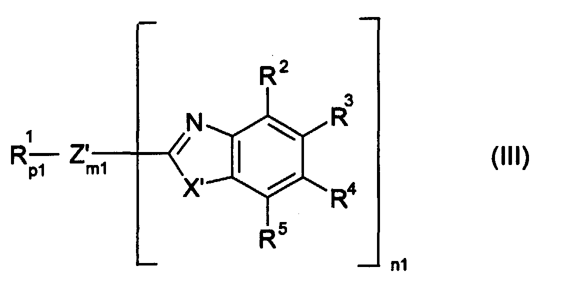 dans laquelle:
- Z 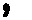 représente un reste organique de valence (p₁ + n₁) comportant une ou plusieurs doubles liaisons placées de telle sorte qu'elle complète le système de doubles liaisons d'au moins deux groupes benzoazolyle tels que définis à l'intérieur des crochets pour former un ensemble totalement conjugué;
- X 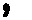 désigne S, O ou NR⁶
- R¹ désigne hydrogène, alkyle en C₁-C₁₈, alcoxy en C₁-C₄, un aryle en C₅-C₁₅, un acyloxy en C₂-C₁₈, SO₃Y 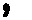 ou COOY 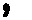 ;
- les radicaux R², R³, R⁴ et R⁵, identiques ou différents, désignent un groupe nitro ou ont les mêmes significations que R¹;
- R⁶ désigne hydrogène, un alkyle en C₁-C₄ ou un hydroxyalkyle en C₁-C₄;
- Y 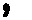 désigne hydrogène, Li, Na, K, NH₄, 1/2Ca, 1/2Mg, 1/3Al ou un cation résultant de la neutralisation d'un groupe acide libre par une base azotée organique;
- m₁ est 0 ou 1;
- n₁ est un nombre de 2 à 6;
- p₁ est un nombre de 1 à 4;
- sous réserve que p₁ + n₁ ne dépasse pas la valeur 6.

Les composés de formule (III) conformes à l'invention sont des filtres UV-A hydrosolubles déjà connus dans la demande de brevet EP-A-0669323 Ils sont décrits et préparés selon les synthèses indiquées dans le brevet US 2,463,264 ainsi que la demande de brevet EP-A-0669323 (ces deux documents faisant partie intégrante du contenu de la description).

Parmi les composés de formule (III) de l'invention, on préfère ceux pour lesquels le groupe Z 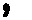 est choisi dans le groupe constitué par:
(a) un radical hydrocarboné en C₂-C₆ aliphatique linéaire insaturé pouvant être interrompu par un groupe aryle en C₅-C₁₂ ou un hétéroaryle en C₄-C₁₀ comme par exemple:
   -CH=CH-, -CH=CH-CH=CH- ou bien 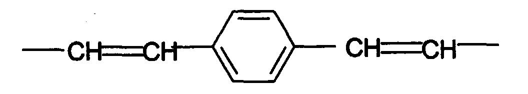
(b) un groupe aryle en C₅-C₁₅ pouvant être interrompu par un radical hydrocarboné en C₂-C₆ aliphatique linéaire insaturé comme par exemple les groupes suivants: 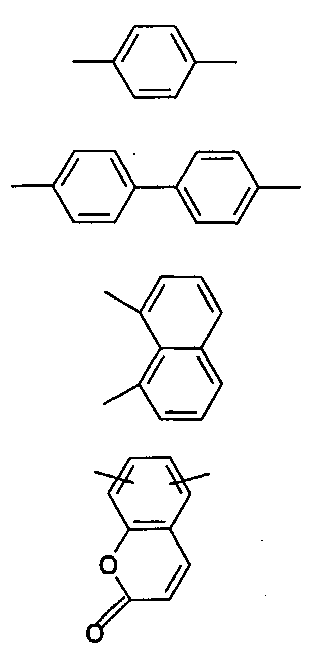 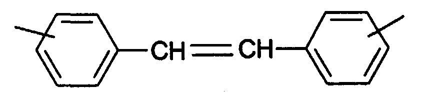
(c) un reste hétéroaryle en C₃-C₁₀ comme par exemple les groupes suivants: 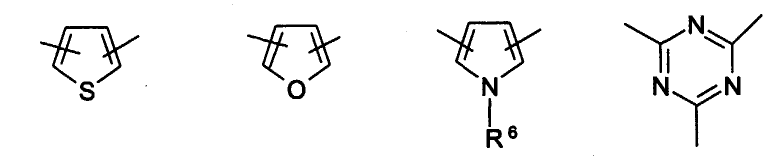 où R⁶ a la même signification indiquée ci-dessus; lesdits radicaux Z 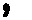 tels que définis dans les paragraphes (a), (b) et (c ) pouvant être substitués par des radicaux alkyle en C₁-C₆, alcoxy en C₁-C₆, phénoxy, hydroxy, méthylènedioxy ou amino éventuellement substitués par un ou 2 radicaux alkyle en C₁-C₅.

A titre d'exemples de composés de formule (III) utilisables, on peut citer ceux de structure suivante ainsi que leurs sels:

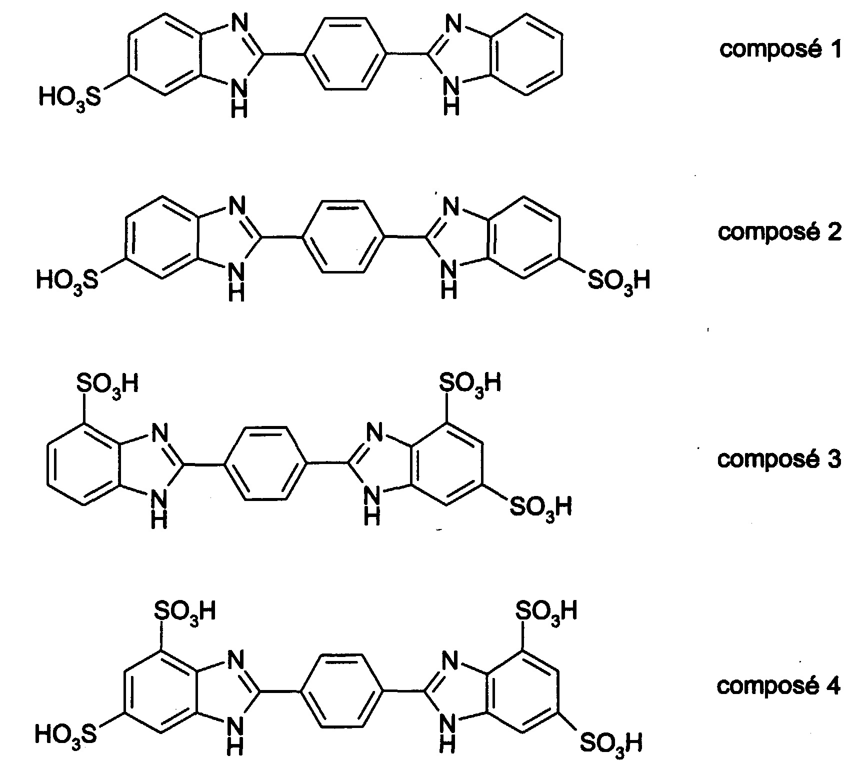

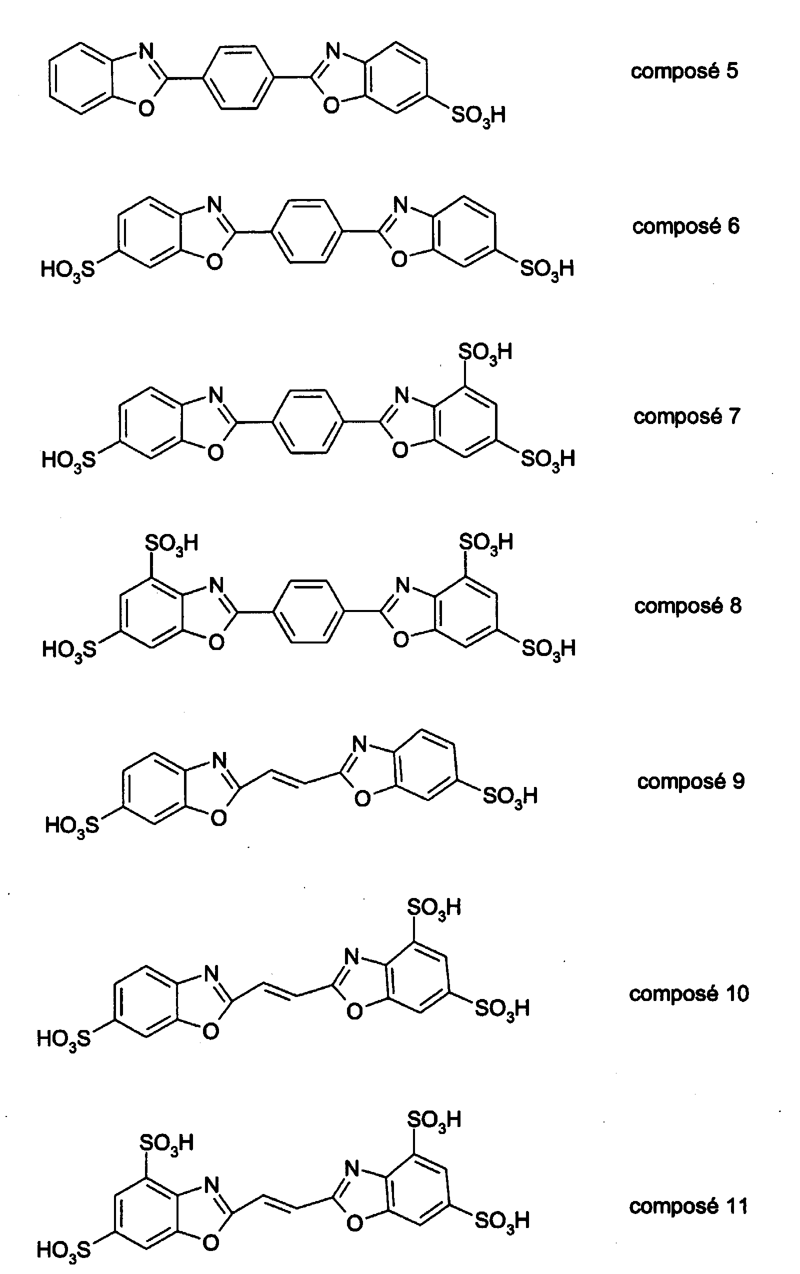

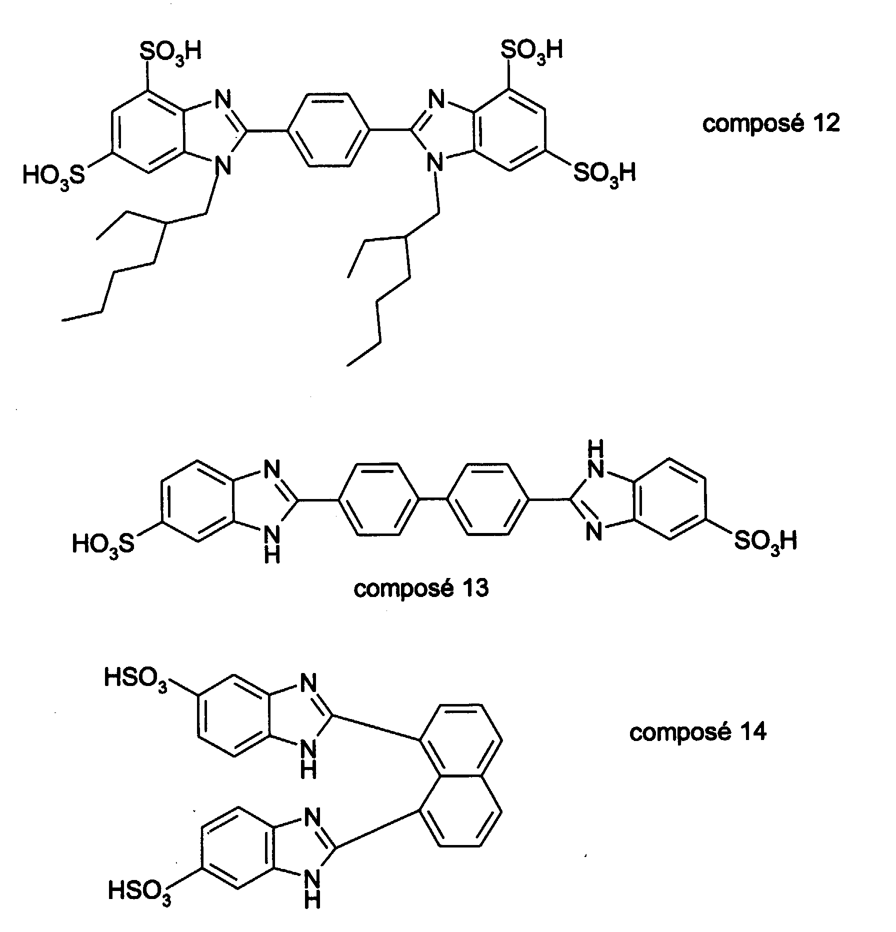

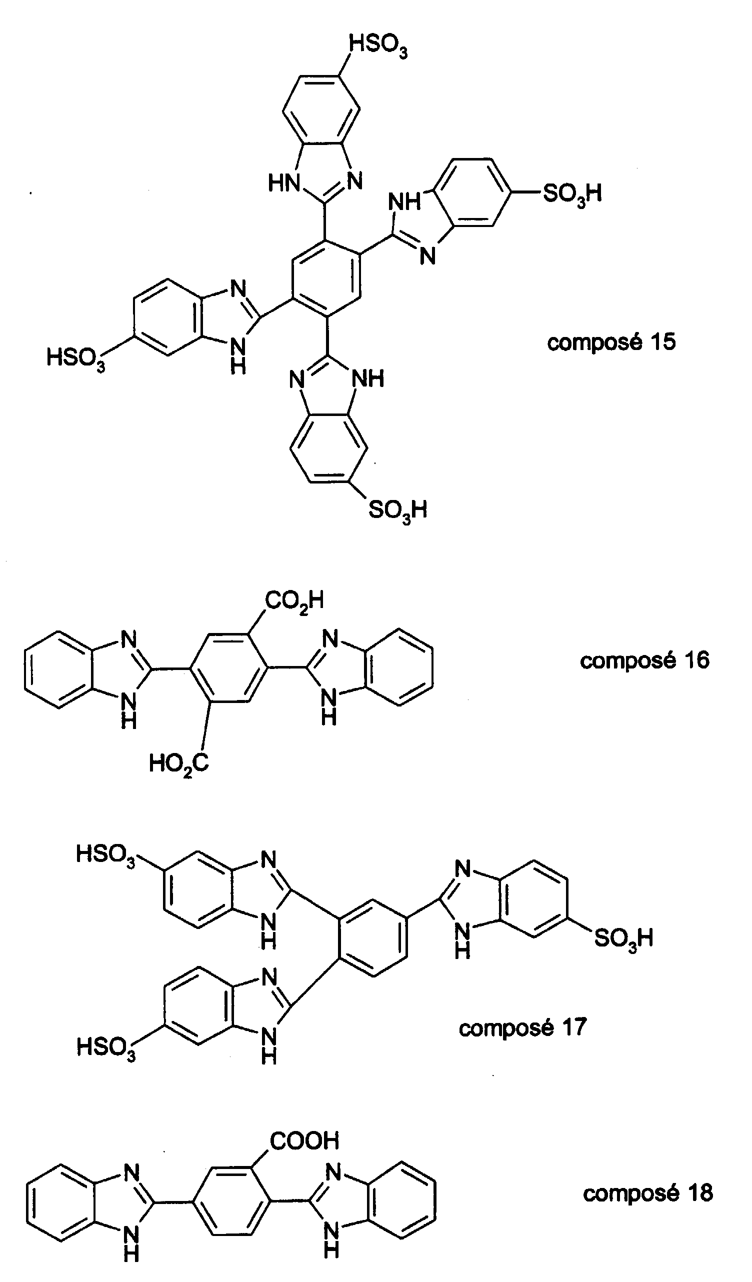

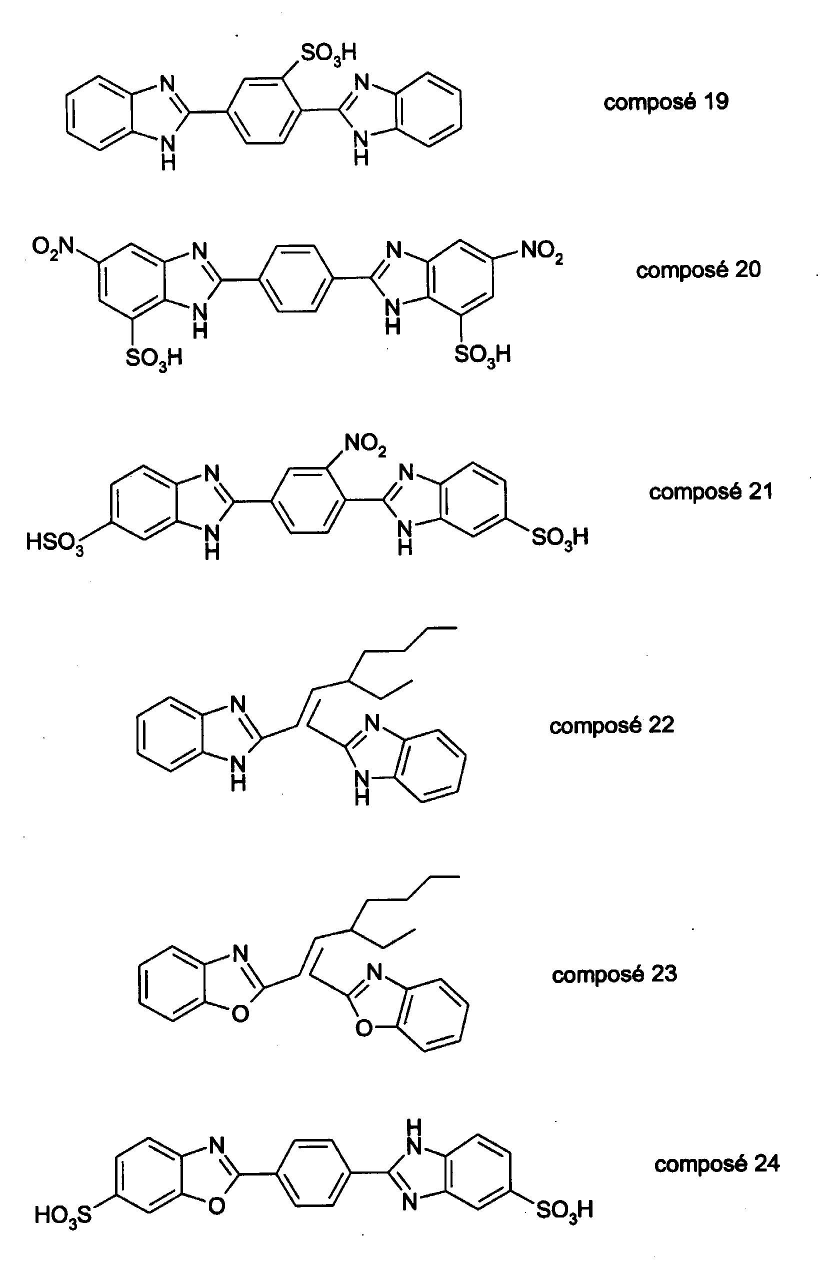

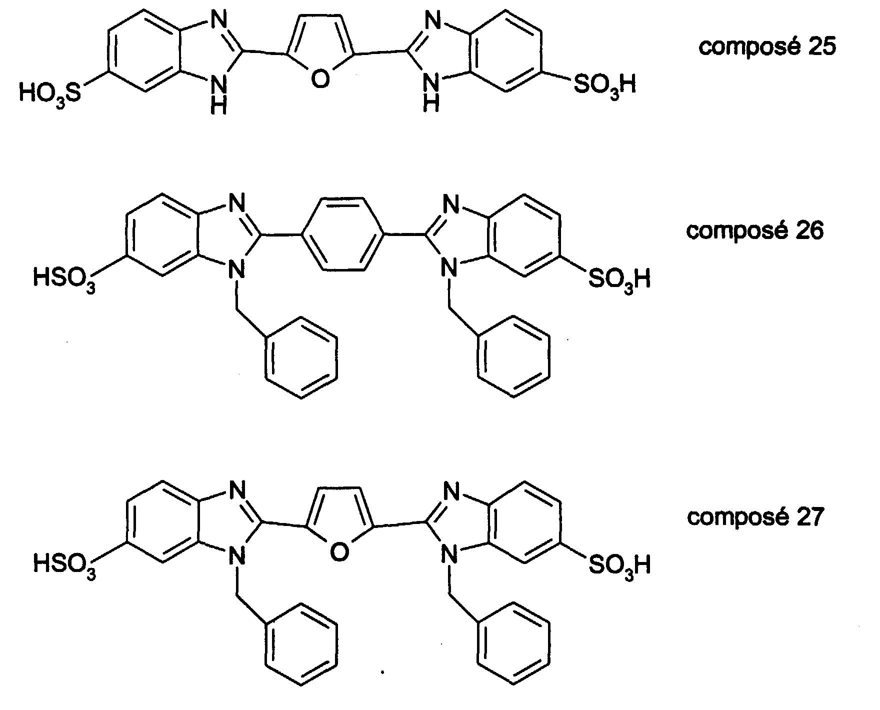

Parmi tous ces composés on préférera tout particulièrement l'acide 1,4-bis-benzimidazolyl-phènylèn-3,3 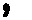 ,5,5 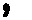 -tétrasulfonique (composé 4) ainsi que ses sels de structure suivante:

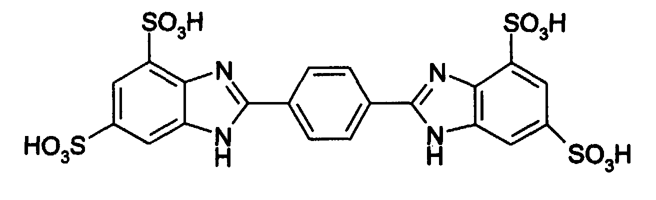

Comme autres exemples de composés comportant au moins deux groupes benzoazolyle, on peut citer également les composés suivants ainsi que leurs sels:

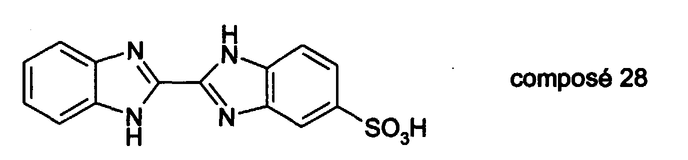

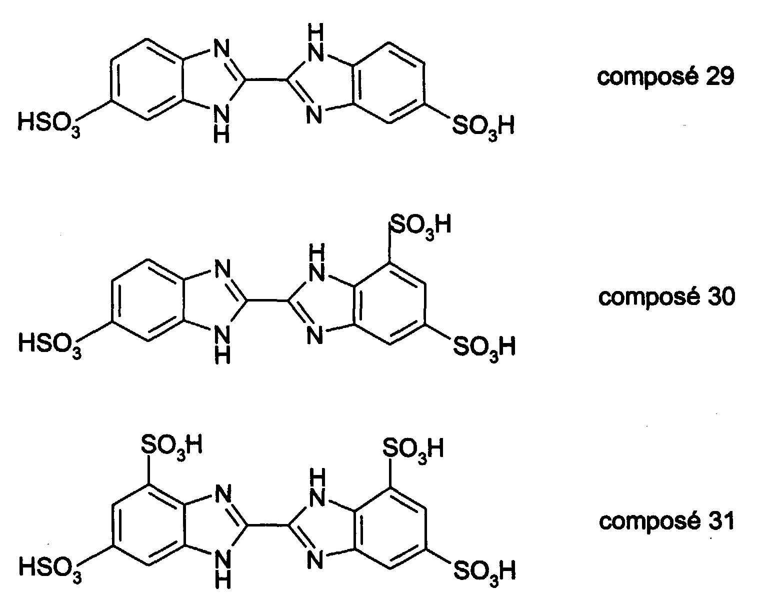

Comme exemples de composés comportant au moins un groupe benzodiazolyle utilisables selon l'invention, on peut mentionner en particulier les composés suivants ainsi que leurs sels:

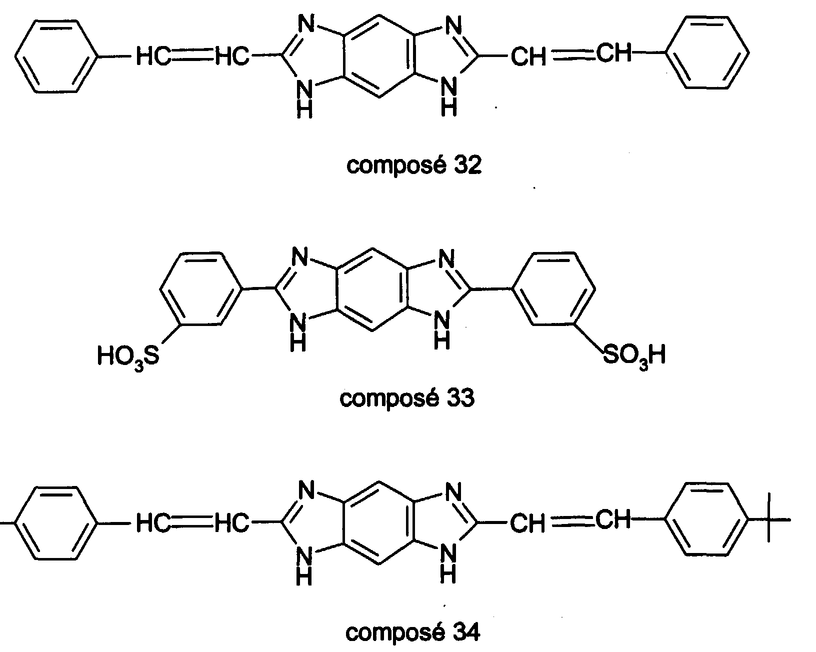

Le ou les composés à groupements benzoazolyle ou benzodiazolyle conformes à l'invention peuvent être présents dans les compositions selon l'invention à une concentration comprise entre 0,1 et 15%, de préférence entre 0,2 et 10%, en poids par rapport au poids total de la composition.

Comme indiqué précédemment, selon une caractéristique essentielle de la présente invention, il convient que les deux types de filtres solaires soient tous deux présents dans la composition finale dans une proportion respective telle qu'un effet de synergie au niveau de l'indice de protection conféré par l'association résultante, soit obtenu de manière notable, substantielle et significative.

En outre, et d'une manière générale, on notera que les concentrations et rapports en dérivés de benzotriazole de formule (I) et en composés à groupements benzoazolyle ou benzodiazolyle tels que définis précédemment sont choisis de manière telle que l'indice de protection solaire de la composition finale soit de préférence d'au moins 2.

Selon un mode préféré de réalisation de la présente invention, le support cosmétiquement acceptable dans lequel se trouvent contenus les différents types de filtres est une émulsion de type huile-dans-eau.

Les compositions cosmétiques antisolaires selon l'invention peuvent bien entendu contenir un ou plusieurs filtres solaires complémentaires actifs dans l'UVA et/ou l'UVB (absorbeurs), hydrophiles ou lipophiles, autres bien sûr que les deux filtres mentionnés ci-avant. Ces filtres complémentaires peuvent être notamment choisis parmi les dérivés cinnamiques; les dérivés salicyliques, les dérivés du camphre; les dérivés de triazine tels que ceux décrits dans les demandes de brevet EP863145, EP517104, EP570838, EP796851, EP775698 et EP878469 ; les dérivés de la benzophénone; les dérivés du dibenzoylméthane; les dérivés de β, β 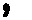 -diphénylacrylate, les dérivés de benzimidazole; les dérivés de l'acide p-aminobenzoïque; les polymères filtres et silicones filtres tels que ceux décrits dans la demande WO-93/04665.

Comme exemples de filtres solaires complémentaires actifs dans l'UV-A et/ou l'UV-B, on peut citer:
l'acide p-aminobenzoïque,
le p-aminobenzoate oxyéthyléné (25mol),
le p-diméthylaminobenzoate de 2-éthylhexyle,
le p-aminobenzoate d'éthyle N-oxypropyléné
le p-aminobenzoate de glycérol,
le salicylate d'homomenthyle,
le salicylate de 2-éthylhexyle,
le salicylate de triéthanolamine,
le salicylate de 4-isopropylbenzyle,
le 4-ter-butyl-4 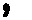 -méthoxy-dibenzoylméthane,
le 4-isopropyl-dibenzoylméthane,
le 4-méthoxy cinnamate de 2-éthylhexyle,
le diisopropyl cinnamate de méthyle,
le 4-méthoxy cinnamate d'isoamyle,
le 4-méthoxy cinnamate de diéthanolamine,
l'anthranilate de menthyle,
le 2-éthylhexyl-2-cyano-3,3 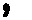 -diphénylacrylate,
l'éthyl-2-cyano-3,3 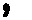 -diphénylacrylate,
l'acide 2-phényl benzimidazole 5-sulfonique et ses sels,
le 3-(4 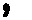 -triméthylammonium)-benzylidèn-bornan-2-on-méthylsulfate,
le 2-hydroxy-4-méthoxybenzophénone,
le 2-hydroxy-4-méthoxybenzophénone-5-sulfonate,
le 2,4-dihydroxybenzophénone,
le 2,2 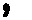 ,4,4 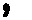 -tétrahydroxybenzophénone,
le 2,2 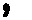 -dihydroxy-4,4 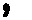 diméthoxybenzophénone,
le 2-hydroxy-4-n-octoxybenzophénone,
le 2-hydroxy-4-méthoxy-4 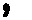 -méthylbenzophénone,
l'acide α-(2-oxoborn-ylidène)-tolyl-4-sulfonique et ses sels
le 3-(4 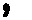 -sulfo)benzylidèn-bornan-2-one et ses sels,
le 3-(4 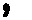 mèthylbenzylidène)-d,l-camphre,
le 3-benzylidène-d,l-camphre,
l'acide benzène 1,4-di(3-méthylidène-10-camphosulfonique) et ses sels,
l'acide urocanique,
la 2,4,6-tris-[p-(2 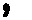 -éthylhexyl-1 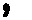 -oxycarbonyl)anilino]-1,3,5-triazine,
2-[(p-(tertiobutylamido)anilino]-4,6-bis-[(p-(2 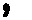 -éthylhexyl-1 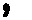 -oxycarbonyl)anilino]-1,3,5-triazine,
la 2,4-bis {[4-2-éthyl-hexyloxy)]-2-hydroxy]-phenyl}-6-(4-méthoxy-phenyl)-1,3,5-triazine;
le polymère de N-[(2 et 4)-[(2-oxoborn-3-ylidèn)méthyl] benzyl]-acrylamide,
les polyorganosiloxanes à fonction malonate.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions de l'invention peuvent comprendre en outre des adjuvants cosmétiques classiques notamment choisis parmi les corps gras, les solvants organiques, les épaississants, les adoucissants, les antioxydants, les opacifiants, les stabilisants, les émollients, les hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les homopolymères d'acide acrylique réticulés, les gommes de guar et celluloses modifiées ou non telles que la gomme de guar hydroxypropylée, la méthylhydroxyéthylcellulose, l'hydroxypropylméthyl cellulose ou encore l'hydroxyéthylcellulose.

Bien entendu, l'homme de l'art veillera à choisir ce ou ces éventuels composés complémentaires et/ou leurs quantités de manière telle que les propriétés avantageuses, en particulier le niveau de photoprotection, attachées intrinsèquement à l'association binaire conforme à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de poudre , de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Des exemples concrets, mais nullement limitatifs, illustrant l'invention, vont maintenant être donnés.

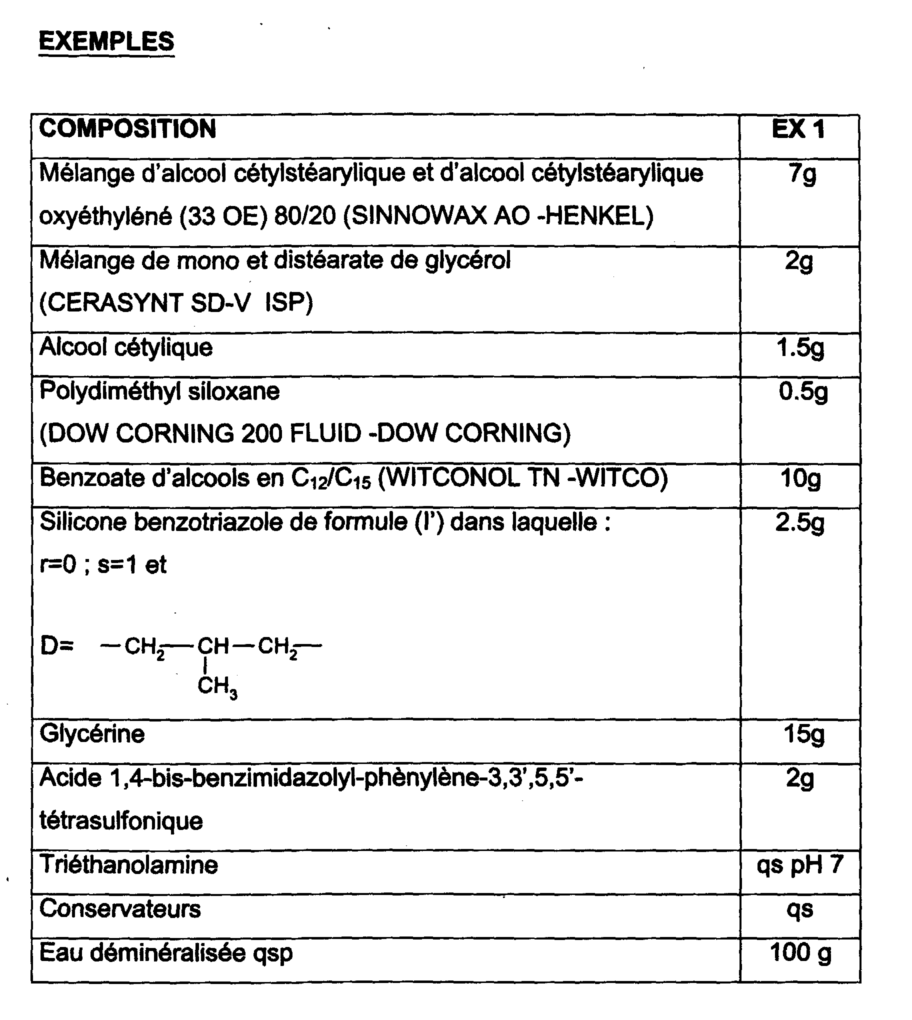

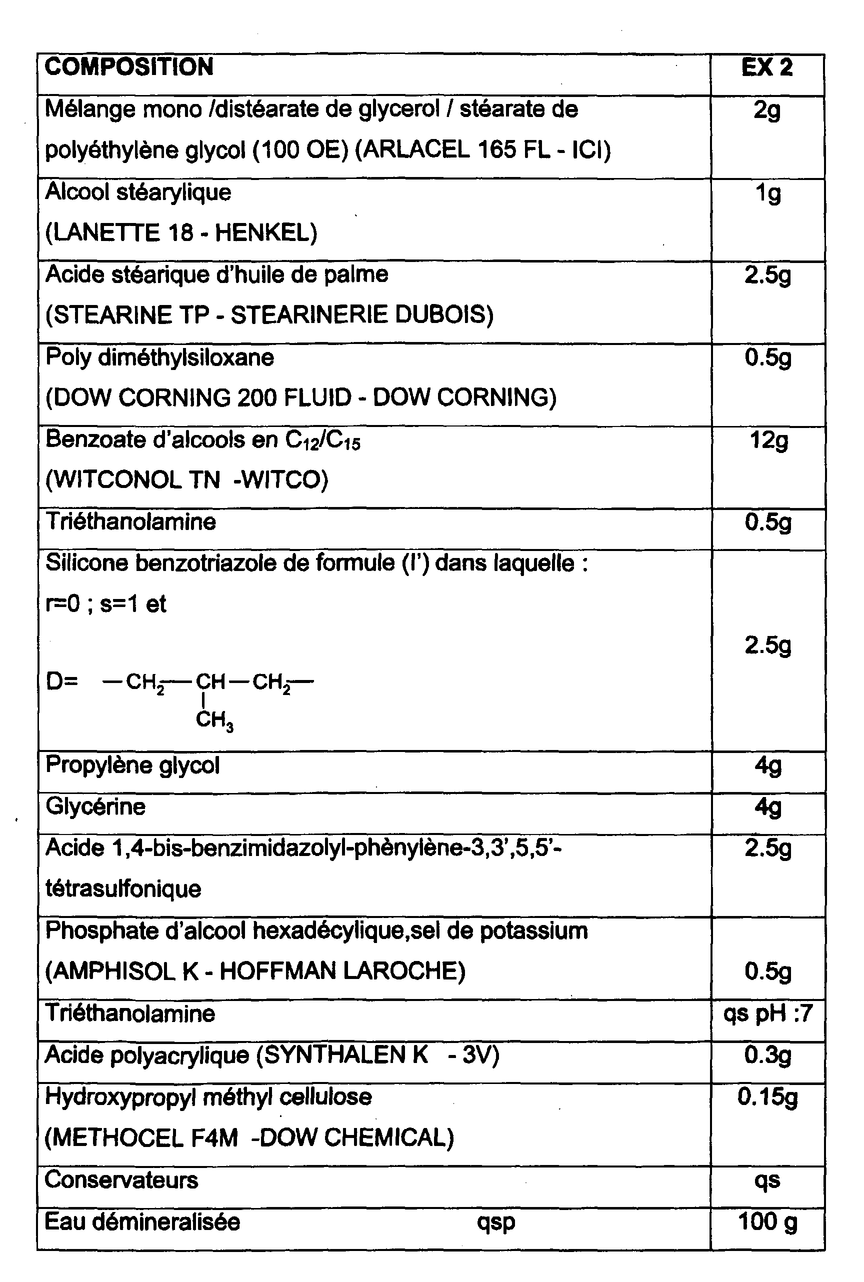

## Revendications

1. Composition cosmétique à usage topique, en particulier pour la photoprotection de la peau et/ou des cheveux, caractérisée par le fait qu'elle comprend, dans un support cosmétiquement acceptable:
(a) à titre de premier filtre, au moins un dérivé de benzotriazole de formule (I) suivante: 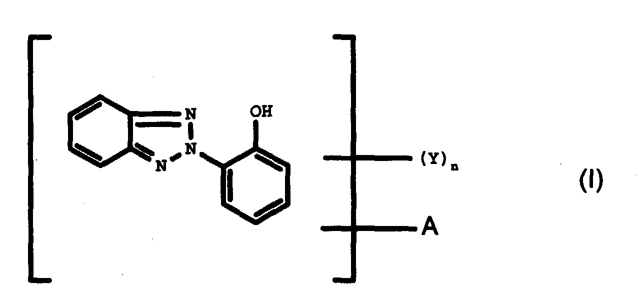 dans laquelle:
- A désigne hydrogène ou un radical divalent -L-W-
- n vaut 1, 2 ou 3;
- Y, identiques ou différents, sont choisis parmi les radicaux alkyles en C₁-C₁₀, les halogènes, les radicaux alkoxy en C₁-C₁₀, des groupes sulfoniques, étant entendu que, dans ce dernier cas, deux Y adjacents d'un même noyau aromatique peuvent former ensemble un groupement alkylidène dioxy dans lequel le groupe alkylidène contient de 1 à 2 atomes de carbone; sous réserve que les radicaux Y soient différents d'un groupe sulfonique quand A est différent d'hydrogène;
- L est un radical divalent de formule (II) suivante: 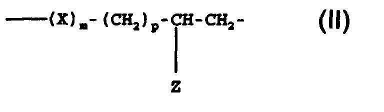 dans laquelle:
- X représente O ou NH,
- Z représente l'hydrogène ou un radical alkyle en C₁-C₄,
- n est un nombre entier compris entre 0 et 3 inclusivement,
- m est 0 ou 1,
- p représente un nombre entier compris entre 1 et 10, inclusivement.
- W est un radical de formule (1), (2) ou (3) suivante: 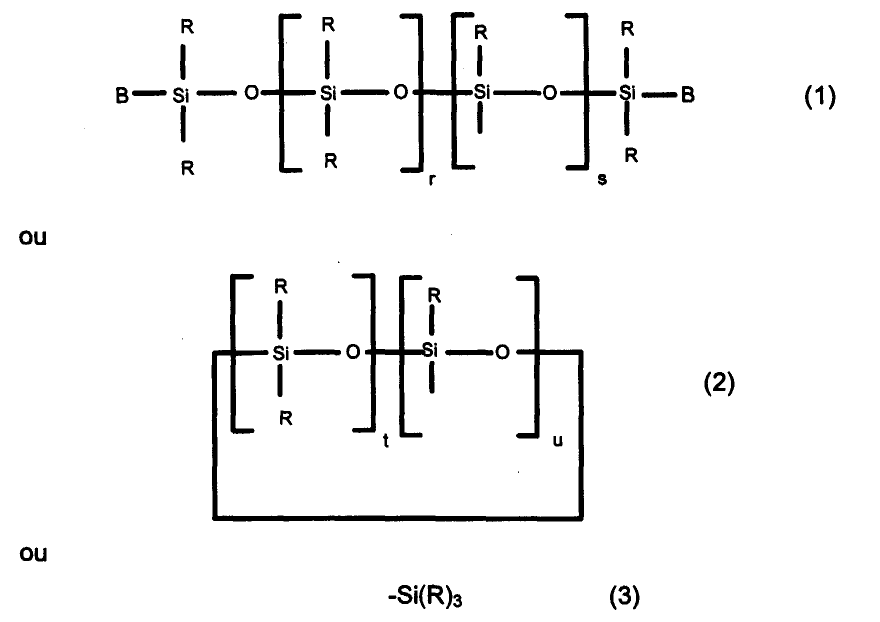 dans lesquelles:
- R, identiques ou différents, sont choisis parmi les radicaux alkyles en C₁-C₁₀, phényle et trifluoro-3,3,3 propyle, au moins 80% en nombre des radicaux R étant méthyle,
- B, identiques ou différents, sont choisis parmi les radicaux R et le radical V de formule suivante: 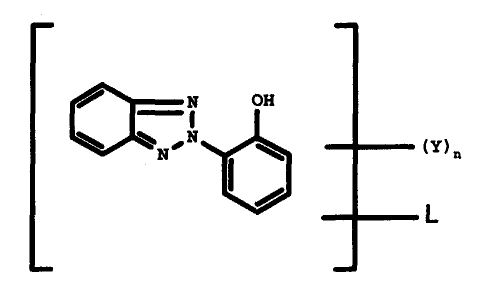 dans laquelle Y, n et L ont les mêmes significations indiquées ci-dessus;
- r est un nombre entier compris entre 0 et 50 inclusivement, et s est un nombre entier compris entre 1 et 20 inclusivement, et si s=0 au moins l'un des deux radicaux B désigne V;
- u est un nombre entier compris entre 1 et 6 inclusivement, et t est un nombre entier compris entre 0 et 10 inclusivement, étant entendu que t + u est égal ou supérieur à 3.
et (b) à titre de deuxième filtre, au moins un composé comportant par molécule au moins deux groupes benzoazolyle et/ou un composé comportant par molécule au moins un groupe benzodiazolyle;
lesdits premier et second filtres étant présents dans une proportion produisant une activité synergique au niveau des indices de protection solaire conférés.

2. Composition selon la revendication 1, où les composés de formule (I) sont choisis dans le groupe constitué par:
- le 2 (2 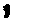 -hydroxy-5 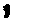 -méthylphényl)benzotriazole;
- le 2 (2 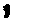 -hydroxy-3 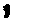 -butyl-5 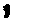 -méthylphényl)benzotriazole;
- le 2 (2 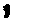 -hydroxy-5 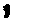 -t-octylphényl)benzotriazole;
- le 2 (2 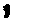 -hydroxy-3 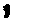 -sec-butyl-5 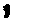 -benzènesulfonate)benzotriazole;

3. Composition selon la revendication 1 où le dérivé de benzotriazole de formule (I) est choisi parmi ceux pour lesquels A désigne un radical divalent -L-W- et W répond à la structure de formule (1) en respectant au moins l'une des caractéristiques suivantes:
- R est alkyle, et de préférence méthyle,
- r est compris entre 0 et 15 inclusivement ; s est compris entre 1 et 5 inclusivement,
- n est non nul, et de préférence égal à 1, et Y est alors choisi parmi méthyle, ter.-butyle ou alcoxy en C₁-C₄,
- Z est hydrogène ou méthyle,
- m=0, ou [m=1 et X=O]
- p est égal à 1.

4. Composition selon la revendication 3, caractérisée par le fait que ledit dérivé de benzotriazole de formule (I) présente l'ensemble desdites caractéristiques.

5. Composition selon la revendication 3 ou 4, caractérisée par le fait le dérivé de benzotriazole est choisi parmi les composés de formule générale (I 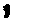 ): 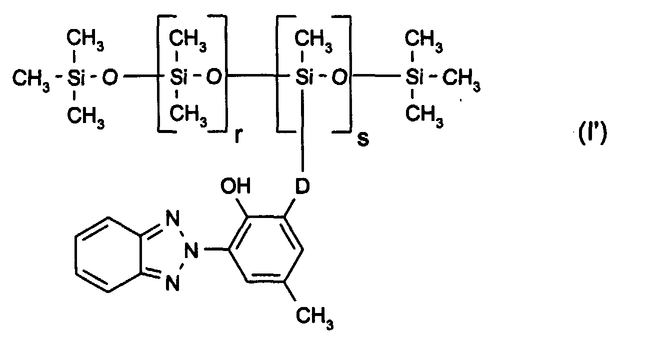 avec
0 ≤ r ≤ 15
1 ≤ s ≤ 5
et où D représente le radical divalent:

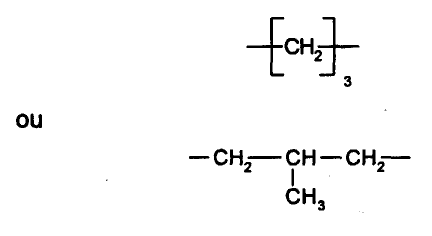

6. Composition selon la revendication 5, caractérisée par le fait que le dérivé de benzotriazole répond à la formule générale (I 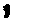 ) dans laquelle:

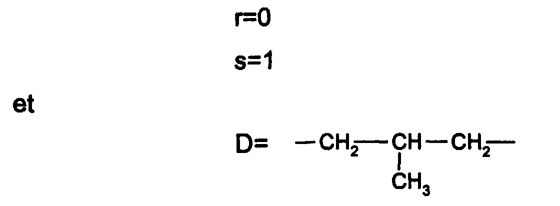

7. Composition selon la revendication 5, caractérisée par le fait que le dérivé de benzotriazole répond à la formule générale (I 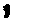 ) dans laquelle:

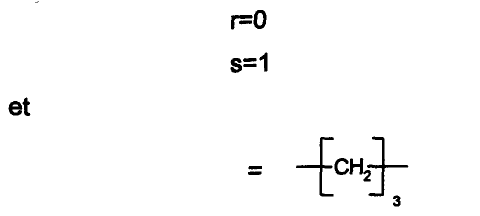

8. Composition selon l'une quelconque des revendications précédentes, caractérisée par le fait que la concentration en dérivé de benzotriazole de formule (I) est comprise entre 0,1 et 15 % en poids par rapport au poids total de la composition.

9. Composition selon la revendication 8, caractérisée par le fait que ladite concentration est comprise entre 0,2 et 10 % en poids par rapport au poids total de la composition.

10. Composition selon l'une quelconque des revendications précédentes, caractérisée par le fait que le composé comportant au moins deux groupes benzoazolyle répond à la formule générale (III) suivante: 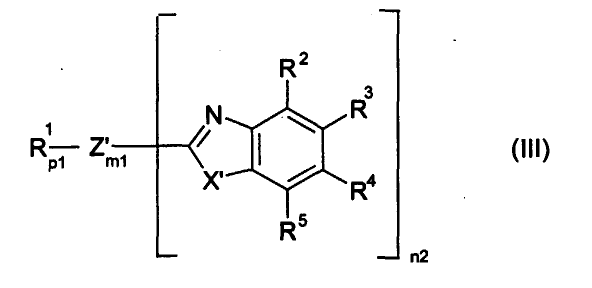 dans laquelle:
- Z 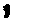 représente un reste organique de valence (p₁ + n₁) comportant une ou plusieurs doubles liaisons placées de telle sorte qu'elle complète le système de doubles liaisons d'au moins deux groupes benzoazolyle tels que définis à l'intérieur des crochets pour former un ensemble totaelement conjugué;
- X 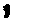 désigne S, O ou NR⁶
- R¹ désigne hydrogène, alkyle en C₁-C₁₈, alcoxy en C₁-C₄, un aryle en C₅-C₁₅, un acyloxy en C₂-C₁₈, SO₃Y 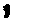 ou COOY 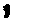 ;
- les radicaux R², R³, R⁴ et R⁵, identiques ou différents, désignent un groupe nitro ou ont les mêmes significations que R¹;
- R⁶ désigne hydrogène, un alkyle en C₁-C₄ ou un hydroxyalkyle en C₁-C₄;
- Y 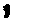 désigne hydrogène, Li, Na, K, NH₄, 1/2Ca, 1/2Mg, 1/3Al ou un cation résultant de la neutralisation d'un groupe acide libre par une base azotée organique;
- m₁ est 0 ou 1;
- n₁ est un nombre de 2 à 6;
- p₁ est un nombre de 1 à 4;
- sous réserve que p₁ + n₁ ne dépasse pas la valeur 6.

11. Composition selon la revendication 10, où le radical Z 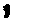 est choisi dans le groupe constitué par:
(a) un radical hydrocarboné en C₂-C₆ aliphatique linéaire insaturé pouvant être interrompu par un groupe aryle en C₅-C₁₂ ou un hétéroaryle en C₄-C₁₀
(b) un groupe aryle en C₅-C₁₅ pouvant être interrompu par un radical hydrocarboné en C₂-C₆ aliphatique linéaire insaturé ;
(c ) un groupe hétéroaryle en C₃-C₁₀; ledit radical Z 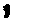 pouvant être substitué par des radicaux alkyle en C₁-C₆, alcoxy en C₁-C₆, phénoxy, hydroxy, méthylènedioxy ou amino éventuellement substitués par un ou 2 radicaux alkyle en C₁-C₅.

12. Composition selon la revendication 10 ou 11, où le radical Z 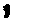 est choisi dans le groupe constitué par les groupes suivants: 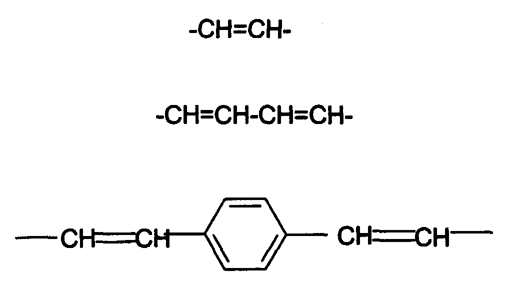 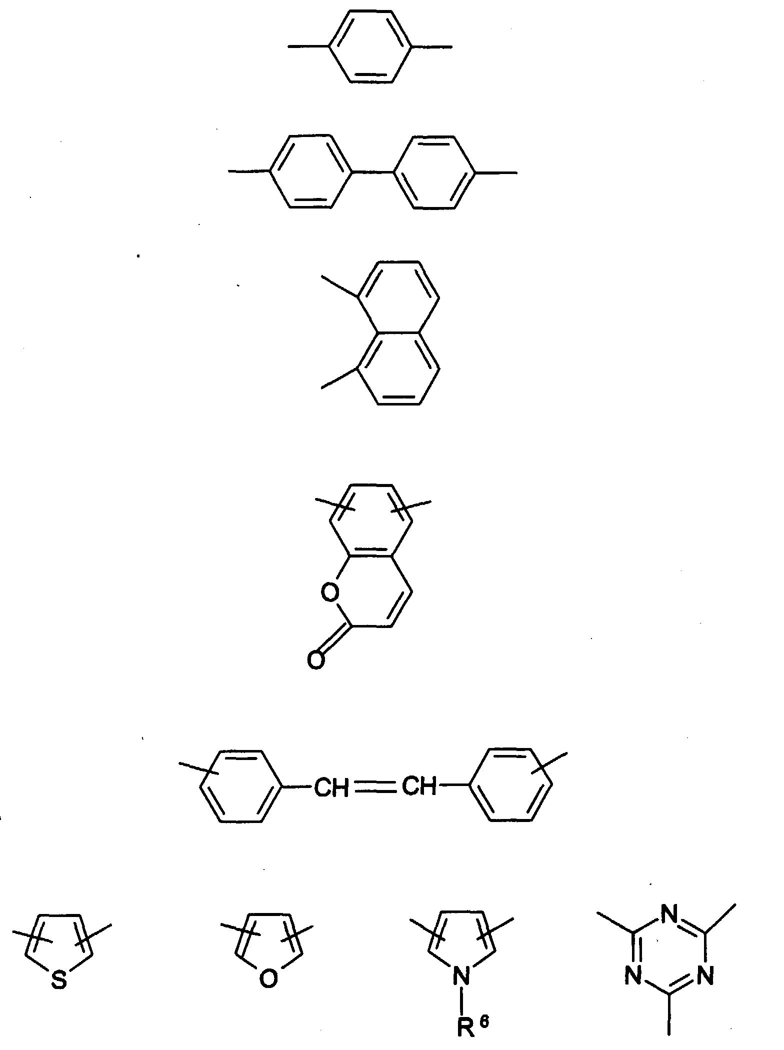 avec R⁶ ayant la même signification indiquée dans la revendication 10.

13. Composition selon l'une quelconque des revendications 10 à 12, où le composé de formule (III) est choisi parmi les composés suivants ou l'un de leurs sels:

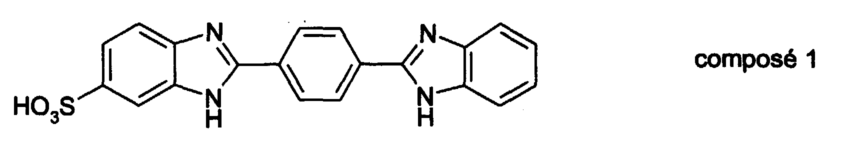

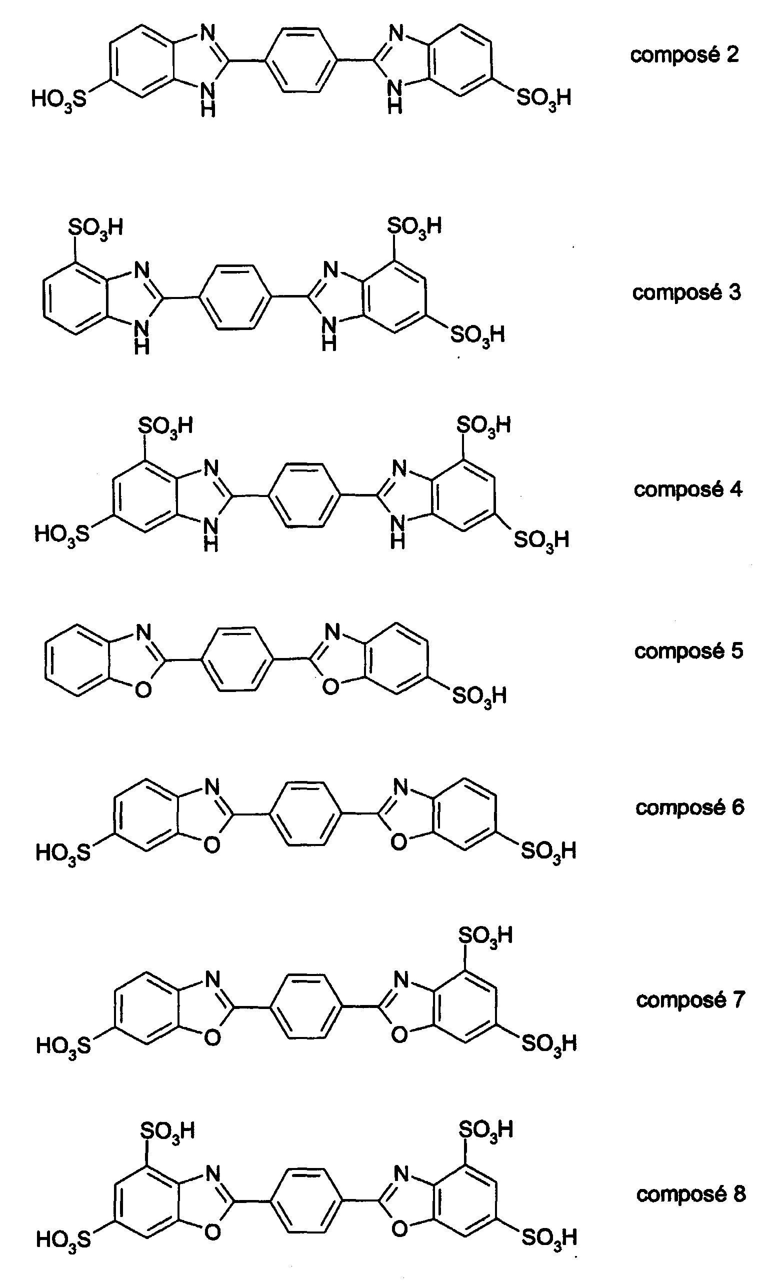

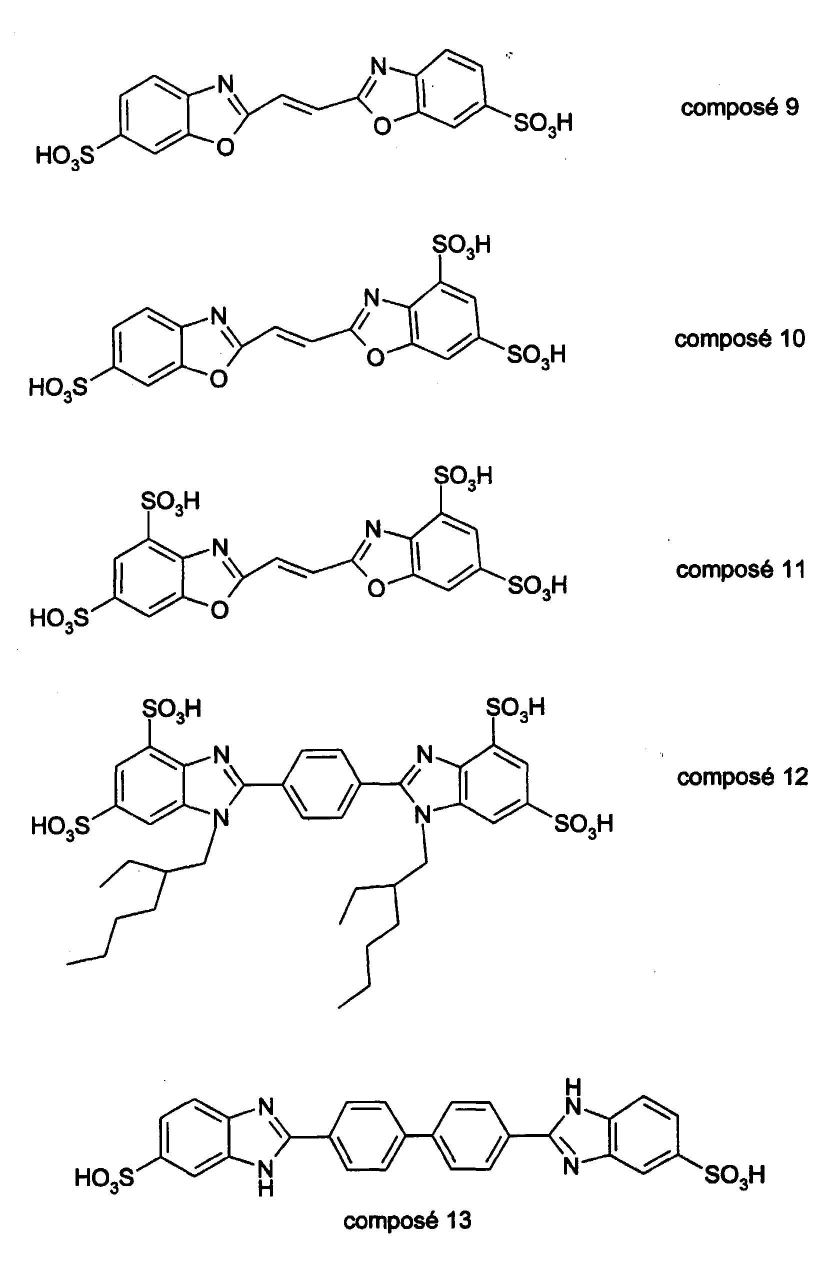

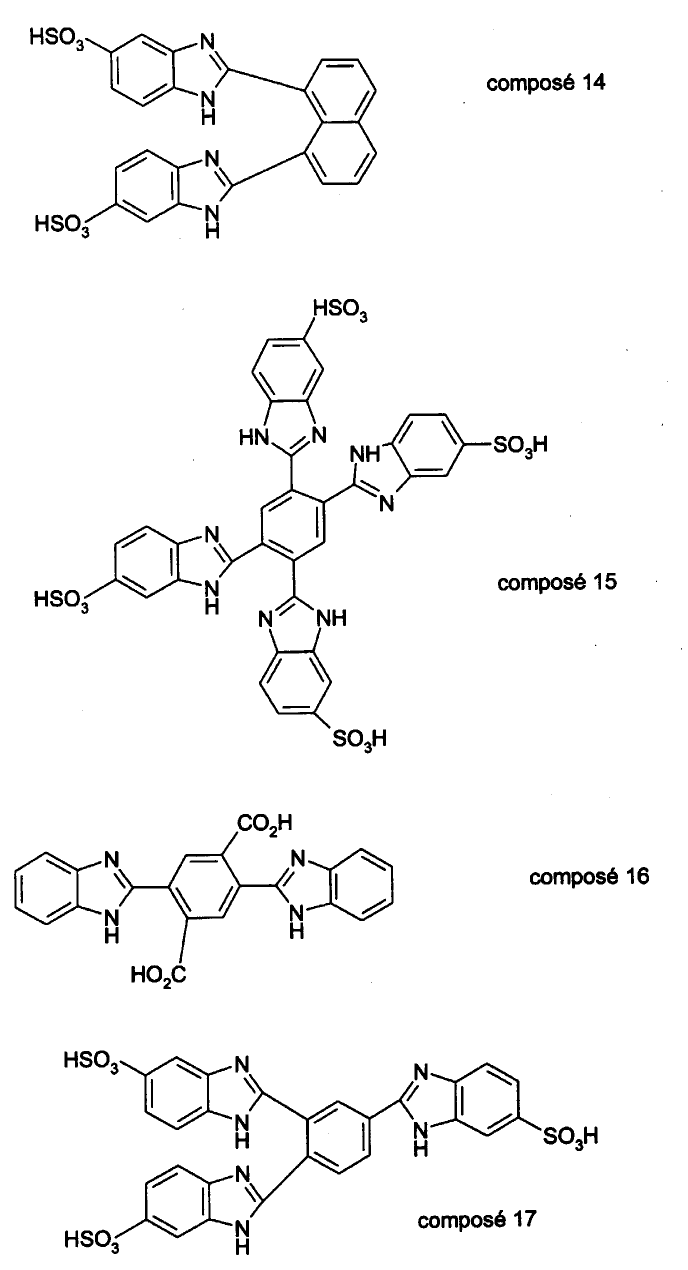

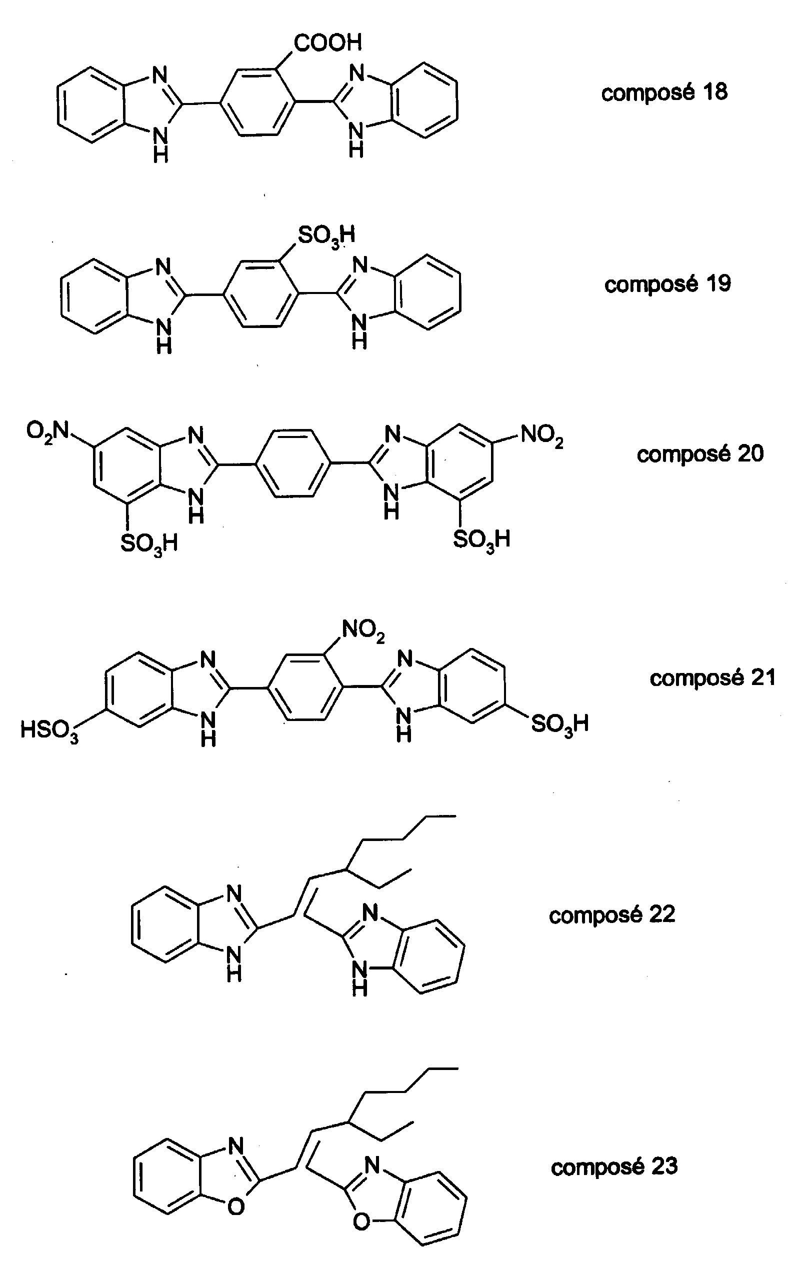

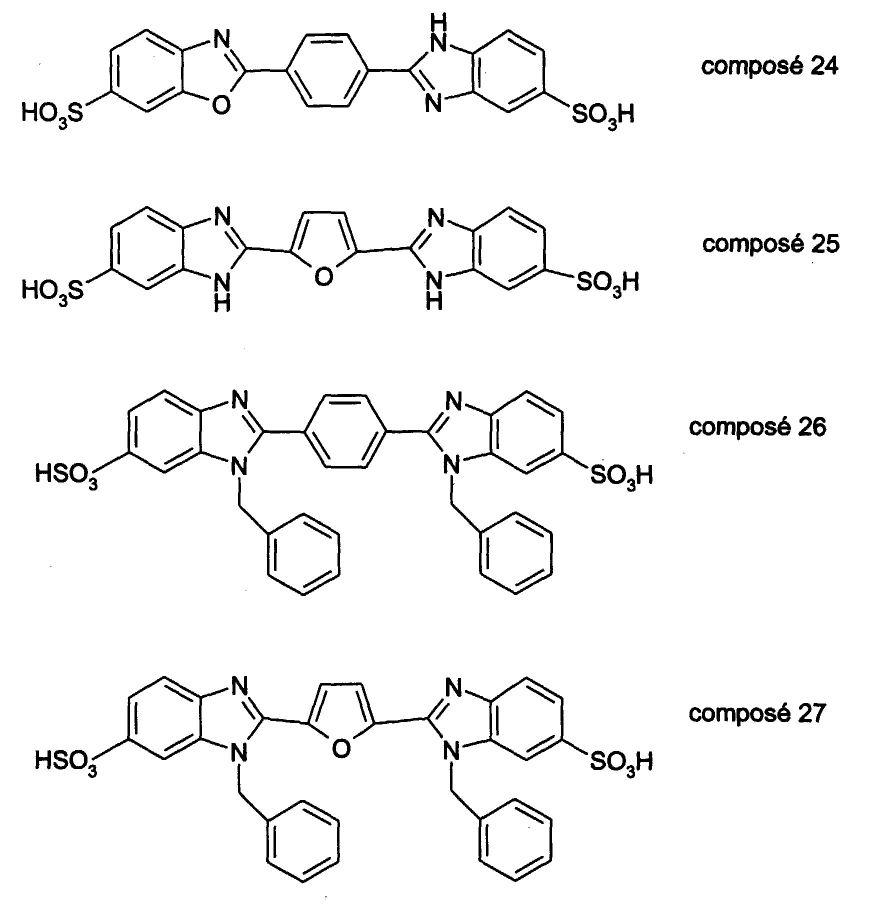

14. Composition selon l'une quelconque des revendications 10 à 13, où le composé de formule (III) est l'acide 1,4-bis-benzimidazolyl-phènylèn-3,3 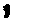 ,5,5 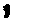 -tétrasulfonique (composé 4) ainsi que ses sels de structure suivante:

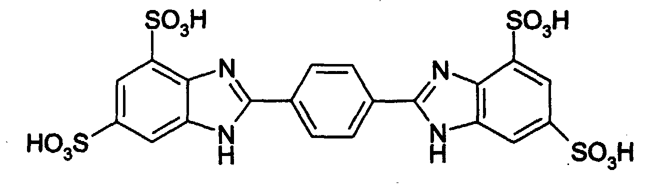

15. Composition selon l'une quelconque des revendications 1 à 9, caractérisée par le fait que le composé comportant au moins deux groupes benzoazolyle est choisi parmi les composés suivants ou l'un de leurs sels:

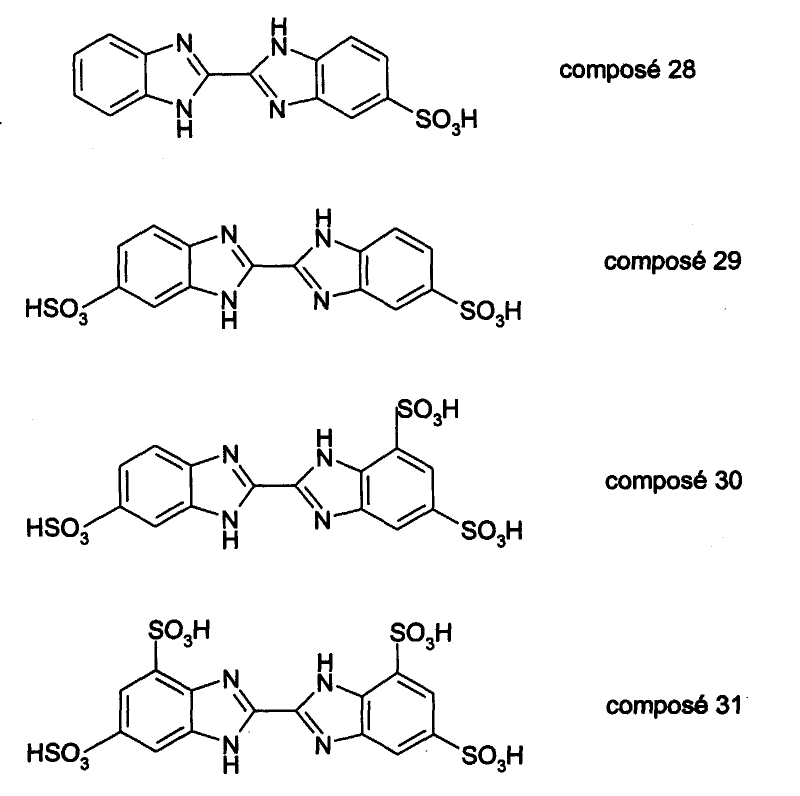

16. Composition selon l'une quelconque des revendications 1 à 9, caractérisée par le fait que le composé comportant au moins un groupe benzodiazolyle est choisi parmi les composés suivants ou l'un de leurs sels:

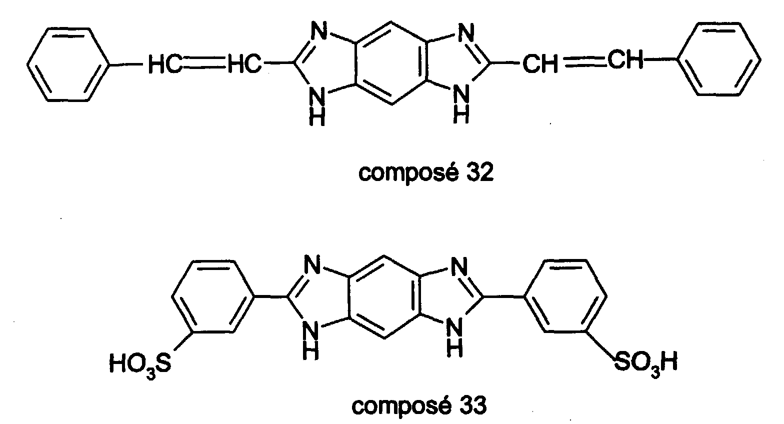

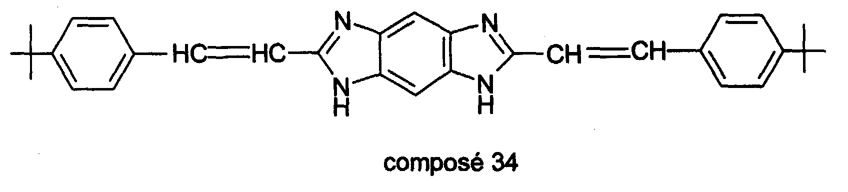

17. Composition selon l'une quelconque des revendications 1 à 16, caractérisée par le fait que le ou les composés à groupements benzoazolyle ou benzodiazolyle sont présents à une concentration comprise entre 0,1 et 15 % en poids par rapport au poids total de la composition.

18. Composition selon la revendication 17, caractérisée par le fait que ladite concentration est comprise entre 0,2 et 10 % en poids par rapport au poids total de la composition.

19. Composition selon l'une quelconque des revendications 1 à 18, caractérisée par le fait que ledit support cosmétiquement acceptable se présente sous la forme d'une émulsion de type huile-dans-eau.

20. Composition selon l'une quelconque des revendications 1 à 19, caractérisée par le fait qu'elle comprend en outre un ou plusieurs filtres organiques complémentaires actifs dans l'UV-A et/ou UV-B, hydrophiles ou lipophiles, différents desdits premier et deuxième filtres.

21. Composition selon la revendication 20, caractérisée par le fait que lesdits filtres organiques complémentaires sont choisis parmi les dérivés cinnamiques, les dérivés salicyliques, les dérivés du camphre, les dérivés de triazine, les dérivés de benzimidazole autres que ceux définis dans les revendications précédentes, les dérivés de la benzophénone, les dérivés du dibenzoylméthane, les dérivés de β,β'-diphénylacrylate, les dérivés de l'acide p-aminobenzoïque, les polymères filtres et silicones filtres.

22. Composition selon l'une quelconque des revendications 1 à 21, caractérisée par le fait qu'elle comprend en outre, à titre d'agents photoprotecteurs UV complémentaires, des pigments ou des nanopigments d'oxydes métalliques, enrobés ou non.

23. Composition selon la revendication 22, caractérisée par le fait que lesdits pigments ou nanopigments sont choisis parmi les oxydes de titane, de zinc, de fer, de zirconium, de cérium et leurs mélanges, enrobés ou non.

24. Composition selon l'une quelconque des revendications 1 à 23, caractérisée par le fait qu'elle comprend en outre au moins un agent de bronzage et/ou de brunissage artificiel de la peau.

25. Composition selon l'une quelconque des revendications 1 à 24, caractérisée par le fait qu'elle comprend en outre au moins un adjuvant choisi parmi les corps gras, les solvants organiques, les épaississants, les adoucissants, les antioxydants, les opacifiants, les stabilisants, les émollients, les hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants.

26. Composition selon l'une quelconque des revendications 1 à 25, caractérisée par le fait qu'il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

27. Composition selon l'une quelconque des revendications 1 à 26, caractérisée par le fait qu'il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

28. Composition selon l'une quelconque des revendications 1 à 26, caractérisée par le fait qu'il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

29. Utilisation de la composition définie à l'une quelconque des revendications 1 à 26 pour la fabrication de, compositions cosmétiques pour la protection de la peau et/ou des cheveux contre le rayonnement ultraviolet, en particulier le rayonnement solaire.

30. Procédé de traitement cosmétique pour protéger la peau et/ou les cheveux contre le rayonnement ultraviolet, en particulier le rayonnement solaire, caractérisé en ce qu'il consiste à appliquer sur ceux-ci une quantité efficace d'une composition telle que définie à l'une quelconque des revendications 1 à 28.
